(19) Europäisches Patentamt
European Patent Office
Office européen des brevets

(11) Veröffentlichungsnummer: **0 166 215**
**B1**

(12) **EUROPÄISCHE PATENTSCHRIFT**

(45) Veröffentlichungstag der Patentschrift: **10.08.88**

(21) Anmeldenummer: **85106361.0**

(22) Anmeldetag: **23.05.85**

(51) Int. Cl.⁴: **C 07 D 225/08**, C 07 B 57/00, C 07 B 55/00 // A61K31/395

# (54) Verfahren zur Herstellung von racemischem Asocainol.

(30) Priorität: **23.05.84 DE 3419099**

(43) Veröffentlichungstag der Anmeldung: **02.01.86 Patentblatt 86/1**

(45) Bekanntmachung des Hinweises auf die Patenterteilung: **10.08.88 Patentblatt 88/32**

(84) Benannte Vertragsstaaten: **AT CH DE FR GB IT LI**

(56) Entgegenhaltungen:
**EP-A-0 035 360**

**THE JOURNAL OF THE AMERICAN CHEMICAL SOCIETY, Band80, 20. Januar 1958, Easton, Pa., USA; J.A. BERSON et al. "Asymmetric Induction Studies with Optically Active Biphenyls. The Reactions of Phenylglyoxylates of the Phenyldihydrothebaine Series with Methylmagnesium Iodide"; Seiten 445-451**
**Arch. Pharm. Suppl. 319 (1982), Nr. 145**

(73) Patentinhaber: **GÖDECKE AKTIENGESELLSCHAFT, Salzufer 16, D-1000 Berlin 10 (DE)**

(72) Erfinder: **Herrmann, Wolfgang, Dr., Zum Baumgarten 13, D-7802 Merzhausen (DE)**
Erfinder: **Satzinger, Gerhard, Dr., Im Mattenbühl 7, D-7809 Denzlingen (DE)**

**EP 0 166 215 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Asocainol [(+)-6,7,8,9-Tetrahydro-2,12,dimethoxy-7-methyl-6(e')-phenethyl-5H-dibenz(d,f) azonin-1-ol] ist ein aus der DE-C-30 07 710 (entspricht EP-A-35 360) bekannter Arzneimittelwirkstoff, der sich hervorragend zur Blockade unerwünschter Reizbildung und Reizleitung im Nervengewebe eignet. Er ist deshalb besonders zur Localanästhesie und zur Therapie von Herzrhythmusstörungen geeignet.

Aufgabe der Erfindung ist es, ein Verfahren zur Herstellung von (±)-Asocainol, bereitzustellen.

Die Aufgabe wurde durch thermische Isomerisierung eines Diastereomeren des (+)-Asocainols zum (-)-Enantiomeren dieses Wirkstoffs und Bildung des Racemats aus den beiden optischen Antipoden gelöst.

Für (+)-Asocainol wurde mittels Röntgenstrukturanalyse eine absolute Konfiguration gemäß Formel (I) ermittelt.

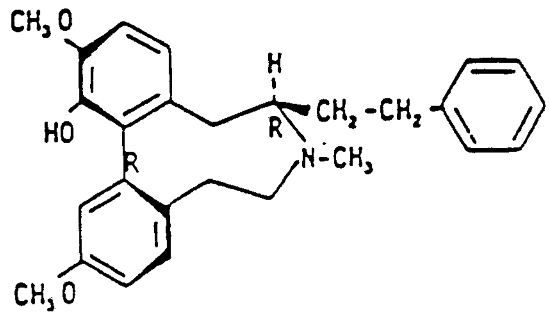

I

Theoretisch kann Asocainol in vier verschiedenen isomeren Formen auftreten, die durch

1.) das asymmetrische C-Atom in Position 6

2.) eine Biphenyl-Asymmetrie an der Bindung C 13a-C 13b

bedingt sind. Die Biphenyl-Asymmetrie wird dadurch hervorgerufen, daß die beiden Phenylringe nicht in einer Ebene liegen, sondern nahezu senkrecht zueinander angeordnet sind. Demnach sind folgende Konfigurationen denkbar.

(R,R) (S,S) (R,S) (S.R)

Die Paare (R,R) und (S,S), sowie die Paare (R,S) und (S,R) sind Enantiomere, die theoretisch zusammen je ein Racemat bilden. Da sich Enantiomere in ihrer Struktur wie Bild und Spiegelbild verhalten, sind sie in allen physikalischen Eigenschaften außer im Vorzeichen der Drehung des polarisierten Lichtes gleich.

Ebenso ist ihr chemisches Verhalten gegenüber achiralen Reagenzien gleich. Mit chiralen Verbindungen reagieren Enantiomere jedoch unterschiedlich, was zu deren Trennung aus racemischen Gemischen ausgenutzt werden kann.

Bei Asocainol tritt neben der optischen "Isomerie" aber auch noch die sogenannte Diastereomerie auf. Diastereomere sind dadurch gekennzeichnet, daß sie keine Bild-Spiegelbild-Beziehung zueinander aufweisen.

So sind die Paare (R,R) und (S,S) diastereomer zu den Paaren (S,R) und (R,S). Ebenso ist das Racemat (R,S; S.S) ein Diastereomeres von Racemat (S,R; R,S). Die bei den Paaren erstgenannte Konfiguration bezieht sich jeweils auf die Biphenyl-Asymmetrie, die zweite auf das asymmetrische C-Atom in Position 6. (S, R) bedeutet: S-Konfiguration bezüglich der Biphenyl-Asymmetrie, R-Konfiguration am C6.

Bei der Synthese von (+)-Asocainol gemäß Beispiel 10 der DE-C-30 07 710 aus natürlichem Thebain (Merck Index 1976 No. 8988) erhält man das Hydrochlorid in einer Ausbeute von 21 % der Theorie. Neben dem gewünschten (+)-Asocainol mit der Konfiguration (R, R) entsteht in dem äußerst ungünstigen Gewichtsverhältnis von 1 : 3,25 das wirkungslose und daher unbrauchbare Diastereomere der Konfiguration (R, S). Alle Versuche, dieses ungünstige Isomerenverhältnis durch Veränderung der Synthese-Parameter, wie z. B. durch Einsatz anderer Lösungsmittel und Variation der Temperatur, der Mengenverhältnisse oder der Konzentration zugunsten einer Ausbeutesteigerung des gewünschten Isomeren zu verschieben, blieben erfolglos. Es wurde jedoch überraschend gefunden, daß sich das unerwünschte Diastereomere (R, S) beim Erhitzen über dessen Schmelzpunkt (138°C) im Biphenyl-System teilweise umlagert, so daß zu 40 % das Enantiomere (S, S), das zu (+)-Asocainol im Bild-Spiegelbild-Verhältnis steht, entsteht. Arbeitet man in einem höhersiedenden organischen inerten Lösungsmittel, so erfolgt eine teilweise Racemisierung bereits ab 130°C. Aber ebensowenig, wie es bisher gelungen ist (+)-Asocainol zu racemisieren, läßt sich das (S,S) Isomere in (+)-Asocainol mit (R, R)-Konfiguration umwandeln. Eine solche Umwandlung ist noch nicht einmal partiell möglich, so daß sich zunächst kein Weg bot, das "falsche" Enantiomere des (+)-Asocainols einer brauchbaren Verwendung zuzuführen. Bei pharmakologischen Untersuchungen stellte sich jedoch unerwartet heraus, daß das (S, S)-Isomere ebenfalls eine antiarrhythmische Wirksamkeit aufweist. Bei der synthetischen Bildung des Racemates (R, R; S, S) aus gleichen Teilen (+)-Asocainol in der (R, R)-Form und (S, S)-Isomeren ergab sich dann ein überraschender pharmakologischer Befund: Anstelle der erwarteten additiven Wirkung wurde ein starker synergistischer antiarrhythmischer Effekt gefunden (vergl. Arch. Pharmacol. Suppl. 319 (1982) Nr. 145; in dieser Veröffentlichung ist nicht angegeben, wie das racemiche Asocainol gewonnen wurde).

Die Erfindung löst das Problem der Verwendung des nicht brauchbaren (R, S)-Isomeren, indem Verfahren angegeben wird nach dem das unerwünschte (R, S)-Isomere in den (S, S)-Isomere ungelagert werden kann, welches mit dem (R, R)-Isomeren ein wirksameres racemisches Asocainol bildet.

Theoretisch waren folgende Wege zur Herstellung des (S, S)-Isomeren denkbar:

1.) Inversion des (R, R)-Isomeren zur (S, S)-Konfiguration

2.) Herstellung durch Grignard-Synthese gemäß DE-C-30 07 710 unter Einsatz von "unnatürlichem" Thebain

Beide Wege erwiesen sich als nicht gangbar. Eine Inversion ist bisher nicht gelungen und "unnatürliches" Thebain als Enantiomeres des natürlichen Thebains steht für ein technisch brauchbares Verfahren nicht zur Verfügung. Die "direkte" Herstellung des Racemats durch Racemisierung von (+)-Asocainol oder durch Grignard-Reaktion aus racemischem Thebain war ebenfalls nicht durchführbar, da racemisches Thebain technisch nicht zugänglich ist und sich (+)-Asocainol mit bisher bekannten Mitteln nicht racemisieren läßt.

Da, vor der Veröffentlichung gemäß Arch. Pharmacol. Suppl 319 (1982) Nr 145 vom Racemat (R, R; S, S) eine im Vergleich zu (+)-Asocainol bessere wirkung nicht erwartet werden konnte, wurden bis zu diesem Zeitpunkt Versuche, das Racemat herzustellen und zu untersuchen nicht unternommen. Erst die unerwartete thermische Umwandlungsmöglichkeit der (R, S)-Konfiguration in die (S, S)-Konfiguration führte zur Entdeckung des überraschenden synergistischen Effekts der zum Racemat vereinigten Asocainol-Enantiomeren (R, R) und S, S), welche in obiger Veröffentlichung publiziert wurde, ohne jeddoch das besondere Verfahren zu seiner Herstellung zu offenbaren.

Im folgenden Formelschema II sind die vier möglichen Konfigurationen und symbolisch deren Racemate wiedergegeben:

II.

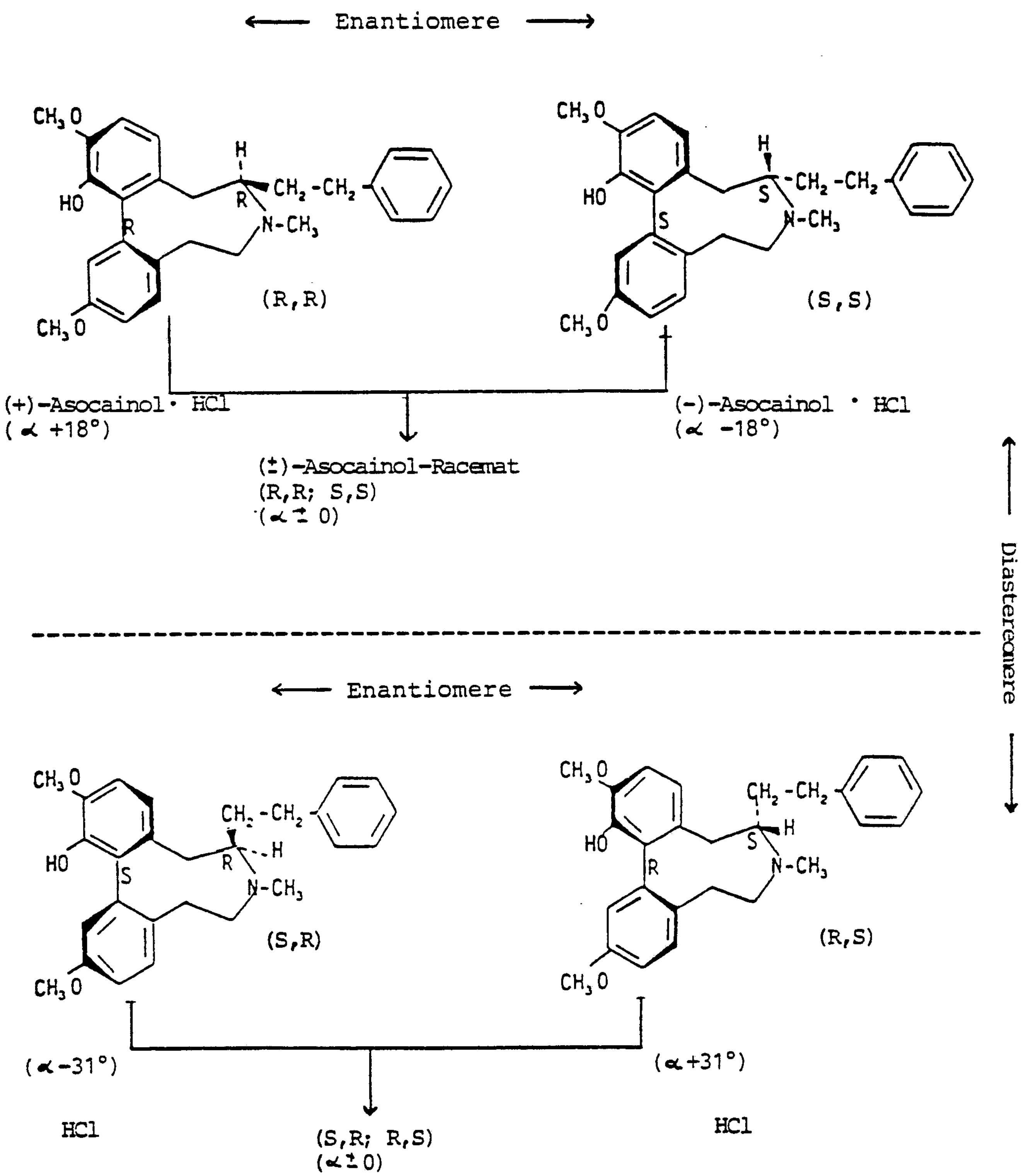


Gegenstand der Erfindung ist ein Verfahren zur Herstellung von racemischem Asocainol durch Grignard-Umsetzung von natürlichem Thebain (The Merck Index 1976, No. 8988) mit Phenethyl-magnesium halogenid, insbesondere dem Bromid oder Chlorid und Trennung der erhaltenen Diastereomeren (R,R) und (R,S) in an sich

bekannter Weise durch fraktionierte Kristallisation der Hydrochloride aus einem niederen Alkohol bevorzugt Isopropanol, das dadurch gekennzeichnet ist, daß man das isolierte Diastereomere (R,S) durch Erhitzen auf 138-200°C oder als Lösung, in einem höher siedenden inerten Lösungsmittel bei 130° bis 200°C teilweise in die (S,S)-Form umlagert, das umgelagerte (S,S)-Enantiomere in an sich bekannter Weise vom unveränderten (R,S) Diastereomeren abtrennt, das (S,S)-Enantiomere im Molverhältnis 1 : 1 mit (+)-Asocainol der (R,R)-Form bevorzugt als Base oder als Salz in einem Lösungsmittel löst und das gebildete Racemat (R,R; S,S) in Form seiner Base oder von dessen pharmakologisch verträglichen Salzen isoliert.

Der im Oberbegriff beschriebene Teil des Verfahrens ist aus der DE-C-30 07 710 bekannt.

Die thermische Umlagerung des Diastereomeren (R,S) kann entweder ohne Lösungsmittel oder in höher siedenden inerten organischen Lösungsmitteln, wie z. B. in Dimethylformamid, Xylol, Ligroin oder Chlorbenzol durchgeführt werden. In diesem Fall kann die thermische Umlagerung auch wenig unterhalb des Schmelzpunkts z. B. bei 130°C durchgeführt werden. Die Trennung des bei der thermischen Isomerisierung gebildeten Gemisches, bestehend aus der (R,S) und der (S,S)-Form erfolgt durch fraktionierte Kristallisation eines Salzes, vorzugsweise des Hydrochlorides, aus einem organischen Lösungsmittel vorzugsweise einem aliphatischen Ester mit bis zu 10 Kohlenstoffatomen, einem aliphatischen Alkohol mit bis zu 6 Kohlenstoffatomen oder aus Mischungen dieser Lösungsmittel.

Die Herstellung des Racemats des Asocainols erfolgt, indem man gleiche Teile der rechts- und linksdrehenden Enantiomeren (R,R) und (S,S) vorzugsweise in Form der freien Basen in einem inerten Lösungsmittel, vorzugsweise in einem Halogenkohlenwasserstoff wie Methylenchlorid, Chloroform, Tetrachlorkohlenstoff oder Dichloräthylen, einem aliphatischen Alkohol mit bis zu 6 Kohlenstoffatomen, bevorzugt in Äthanol oder Isopropanol, oder einem aliphatischen Ester oder Ether mit bis zu 10 Kohlenstoffatomen, löst und das Racemat entweder durch Zugabe von Säuren, bevorzugt von gasförmigem Chlorwasserstoff, als Salz ausfällt oder durch Abdampfen des Lösungsmittels als freie Base isoliert.

Es besteht aber auch die Möglichkeit, das Racemat durch Abkühlen oder Ausfällen mittels eines Lösungsmittels mit schlechten Lösungseigenschaften für das Racemat, wie z. B. Petrolether oder niedere aliphatische Ether, zu fällen.

Die Überführung der freien Basen des racemischen Asocainols, in dessen pharmakologisch verträgliche Salze erfolgt durch Umsetzung mit einer anorganischen oder organischen Säure, wie z. B. Salzsäure, Schwefelsäure, Phosphorsäure, Bromwasserstoffsäure, Essigsäure, Fumarsäure, Oxalsäure, Milchsäure, Zitronensäure, Äpfelsäure, Salicylsäure, Malonsäure, Maleinsäure, Bernsteinsäure oder Ascorbinsäure. Besonders bevorzugt sind Salzsäure, Phosphorsäure und Zitronensäure. Zur Herstellung von Arzneimitteln werden die Wirkstoffe mit üblichen Zusätzen und flüssigen oder festen Trägerstoffen verarbeitet. Racemisches Asocainol kann in weiten Dosierungsgrenzen in flüssiger oder fester Form oral oder parenteral appliziert werden.

Übliche Zusätze für flüssige Formen sind z. B. Tartrat- und Citrat-Puffer, Äthanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls Geschmacks- und Süßstoffe enthalten.

Die folgenden Beispiele dienen der weiteren Erläuterung der Erfindung.

**Beispiel 1**

A):Herstellung von (+)-6,7,8,9-Tetrahydro-2,12,dimethoxy-7-methyl-6(e')-phenethyl-5H-dibenz (d,f) azonin-1-ol Hydrochlorid [(R, R)-Form] (Ausgangsprodukt (+)-Asocainol); siehe auch DE-C-3 007 710.

In einem 50 l Reaktionsbehälter aus Glas werden nach dem Evakuieren und Spülen der gesamten Apparatur mit Stickstoff 528,0 g (21,7 Mol) Magnesiumspäne und 2,0 l absolutes Tetrahydrofuran vorgelegt. Unter Zusatz von wenig Jod wird das Gemisch auf 50°C erwärmt und 3714,0 g (20,0 Mol) 2710,94 ml Phenethylbromid in 5,0 l Toluol während 2,5 Stunden bei 50-60°C zugegeben.

Nach beendetem Zutropfen wird 30 Minuten bei 50-60°C nachgerührt und anschließend eine Stunde zum Rückfluß erhitzt.

Unter gutem Rückfluß und Rühren werden dann unter Stickstoff 3125,0 g (10,036 Mol) Thebain, bei 95°C gelöst in 24,0 l Toluol, während 20 Minuten zulaufen gelassen. Danach wird zwei Stunden unter Rückfluß nachgerührt.

Nach dem Abkühlen auf 50°C wird das Reaktionsgemisch in 20 l-Wasser und 30 l Eis eingerührt, mit 750 ml Eisessig auf pH 7,0 gestellt und die Phasen getrennt. Die wässrige Phase wird mit insgesamt 45 l Methylenchlorid ausgerührt; die vereinigten organischen Phasen werden mit Wasser gewaschen und anschließend über einem Gemisch von Natriumsulfat und Kieselgel getrocknet. Nach dem Filtrieren über ein Einschichtenfilter wird mit Methylenchlorid nachgewaschen und das Filtrat im Vakuum zur Trockene eingeengt. Als Rückstand erhält man 4065,0 g Rohbase ≙ 97,0 % d. Th., bezogen auf Thebain.

Die Zusammensetzung der Rohbase wird mittels HPLC untersucht. Der Gehalt an gewünschtem Isomeren

beträgt 20-23 %, der Gehalt an diastereomerer Verbindung 65 bis 70 Prozent.

4065,0 g (9,736 Mol) Rohbase werden im 100 l-Behälter bei 60-70° C in 21,68 l Essigester und 3,42 l Isopropanol gelöst und dann mit einer frisch bereiteten Lösung von 0,356 kg (9,74 Mol) Chlorwasserstoff-Gas in 2,0 l Isopropanol das Hydrochlorid der (R, R)-Form ausgefällt (bis pH 2-3). Unter Rühren wird während 1-2 Stunden mittels Kaltwasser auf 17 - 18° C gekühlt und das ausgefallene Kristallisat abfiltriert, mit gekühltem Isopropanol nachgewaschen und im Umlufttrockenschrank bei 70-90° C getrocknet. Man erhält 800 g farbloses (+)-6,7,8,9-Tetrahydro-2,12,dimethoxy-7-methyl-6(e')-phenethyl-5Hdibenz (d,f) azonin-1-ol Hydrochlorid (17,5 % d. Therorie).

Schmp. : 229,5° C; HPLC Gehalt > 99 %; $(\alpha)_D = +17{,}8°$ ($c = 1/H_2O$)

Aus 625 g (25,7 mol) Magnesium und 3353 g (23,8 mol) 2-Phenylethylchlorid wird in 7,5 l Tetrahydrofuran eine Grignardlösung hergestellt. Bei 105-110° C gibt man innerhalb von 30 Minuten eine 90-100° C Warme Lösung aus 3516 g (11,2 mol) Thebain in 30 l Toluol dazu. Die Reaktionsmischung wird noch 1 Stunde auf ca 110°C (schwacher Rücktfluß gehalten, dann auf 40-50° C abgekühlt und in 40 l Wasser gegeben. Nach dem Ansäuern mit Essigsäure (pH 6 - 7) wird die organische Phase abgetrennt und die wässrige Phase mit insgesamt 35 l Toluol extrahiert. Die vereinigten Toluolphasen werden mit Natriumsufat getrocknet und anschließend im Vakuum auf ca. 20 l eingeengt. Bei 50°C wird eine Lösung aus 183 g Chlorwasserstoffgas in 800 ml Isopropylalkohol zugegeben, auf 15°C gekühlt und nach 2,5 Stunden das Kristallisat abzentrifugiert, gewaschen und getrocknet.

Ausbeute: 1,8 kg R,R-Form 32= 35 % der Theorie, Reinheit nach HPLC 91 %

Aus der Mutterlauge werden dem gemäß a) 1,6 kg = 36 % der Theorie der R.S-Verbindung isoliert, HPLC-Gehalt 91 %.

B) Isolierung von (+)-6,7,8,9-Tetrahydro-2,12-dimethoxy-7-methyl-6(a') phenethyl-5H-dibenz [d,f] azonin-1-ol [(R, S)-Form] (Isolierung der (R, S)-Form)

Die Mutterlauge der Hydrochloridfällung gemäß Beispiel 1 wird im Vakuum zur Trockene eingeengt. Man erhält 4270,0 g Rückstand. Dieser wird in 30 l Methylenchlorid, 15 l Wasser und 0,75 l Salzsäure conc. gelöst. Nach kurzem Rühren werden die Phasen getrennt und die wässrige Phase noch 2x mit je 5 l Methylenchlorid ausgerührt. Aus den vereinigten Methylenchloridphasen wird durch Zugabe von 15,0 l Wasser und 1,5 l Ammoniak conc. die Base freigesetzt. Nach dem Abtrennen der organischen Phase wird die wässrige Phase noch 2x mit je 5 l Methylenchlorid ausgerührt und dann verworfen.

Die vereinigten Methylenchloridphasen werden mit 15,0 l Wasser gewaschen und über Natriumsulfat und Kieselgel getrocknet. Nach dem Filtrieren wird das Lösungsmittel unter Vakuum abdestilliert. Der so erhaltene Rückstand wird direkt aus 65,0 l Methanol umkristallisiert. Man läßt über Nacht stehen, kühlt unter langsamem Rühren auf 0° C ab und zentrifugiert. Der Rückstand wird bei 70 - 80° C im Umlufttrockenschrank getrocknet. Als Kristallisat erhält man 1,70 kg fast farbloses (+)-6,7,8,9-Tetrahydro-2,12-dimethoxy-7-methyl-6(a') phen-ethyl-5H-dibenz [d,f] azonin-1-ol (40,5 % d. Theorie). Durch Einengen der Mutterlauge und nochmaliges Umkristallisieren des Zweitkristallisats werden weitere 0,437 kg Base gewonnen (10,3 % d. Theorie). Die Gesamtausbeute beträgt somit 2,137 kg ≙ 50,8 % d. Theorie. Schmp. : 138° C; HPLC-Reinheit > 98 %: $(\alpha)_D = +76°$ (c 1/MeOH)

C) Partielle Umlagerung zu (-)-6,7,8,9-Tetrahydro-2,12-dimethoxy-7-methyl-6(e') phenethyl-5H-dibenz [d.f] azonin-1-ol Hydrochlorid (Partielle umlanerung der (R, S)-Form in die (S, S)-Form).

In einen 10 l-Flanschkolben mit Stickstoff-Anschluß, Kühler, Thermometer, Rührer und Heizpilz werden 2770 g (6,634 Mol) der (R, S)-Base aus Beispiel 1B) mehrere Stunden unter Stickstoff auf eine Temperatur von über 150° C erhitzt. Nach Abkühlen des Reaktionsgemisches auf ca. 100° C werden der Schmelze unter gutem Rühren 6,0 l Essigester zugesetzt. Dabei fällt die Temperatur auf 40° C. Man versetzt die Lösung mit 400 g Kieselgel 60 und 200 g Bleicherde und rührt eine Stunde. Danach wird filtriert, der Filterrückstand mit 10,0 l Essigester nachgewaschen, das gesamte Filtrat mit 6,0 l Essigester und 2,2 l Isopropanol verdünnt und dann mit 10 %-iger Essigester/HCl-Lösung bis pH 2-3 versetzt. Nach dem Abkühlen auf 15° C wird das Kristallisat abgesaugt, mit Essigester nachgewaschen und dann im Umlufttrockenschrank bei 80 - 90° C getrocknet. Man erhält 1037,0 g farbloses (-)-6,7,8,9-Tetrahydro-2,12-dimethoxyl-7-methyl-6(e')-phen-thyl-5H-dibenz [d,f,] azonin-1-ol Hydrochlorid (34,4 % d. Theorie) Schmp.: 229,5°; $(\alpha)_D = -17{,}5°C$ ($c = 1/H_2O$): HPLC-Reinheit > 99 %

Die Mutterlauge der Hydrochloridfällung enthält noch ca. 60 % der eingesetzten (R, S)-Form. Diese Substanz kann zum größten Teil zurückgewonnen werden.

D) Herstellung von (±)-6,7,8,9-Tetrahydro-2,12-dimethoxy-7-methyl-6-(e')phenethyl-5H-dibenz [d,f] azonin-1-ol, (Herstellung von Asocainol-Racemat)

In einem 30 l-Ausrührgefäß werden 1030,0 g (2,268 Mol) der (R, R)-Form (Beispiel 1) und 1030,0 g (2,268 Mol) der (S, S)-Form (Beispiel 3), als Hydrochlorid zusammen in 20,0 l Wasser gelöst. Dann werden 5,0 l Methylenchlorid zugegeben und die Lösung anschließend mit Ammoniak conc. alkalisiert. Die Phasen werden getrennt und die wässrige Phase noch 3x mit je 5,0 l Methylenchlorid ausgerührt. Die vereinigten Methylenchloridauszüge werden mit Wasser gewaschen, über Natriumsulfat und Kieselgel getrocknet und nach dem Filtrieren im Vakuum bei 50° C Badtemperatur zur Trockene eingeengt. Man erhält als Rückstand 1905,0 g racemische Base.

$(\alpha)_D = \pm 0$ (c=1/MeOH)

Die Base wird dann in 18,5 l Essigester und 1,85 l Isopropanol gelöst, über ein Faltenfilter filtriert und mit 10 %-iger Essigester/ HCl-Lösung das Hydrochlorid gefällt (pH 2-3). Nach dem Abkühlen auf 15°C wird abzentrifugiert und im Umlufttrockenschrank bei 80° C getrocknet. Die Ausbeute beträgt 1844,0 g (±)-6,7,8,9-Tetrahydro-2,12-dimethoxy-7-methyl-6-(e') phenethyl-5H-dibenz [d,f] azonin-1-ol Hydrochlorid = racemisches Asocainol HCl.

Schmp.: 214°C; HPLC-Reinheit > 99,5 %; $(\alpha)_D = \pm 0°C$ ($c = 1/H_2O$)

Zur Herstellung der Base wird eine 5-prozentige wäßrige Lösung des Hydrochlorids mit Natronlauge alkalisiert und die gebildete Base mit Dichlormethan oder Chloroform extrahiert. Der nach Abdestillation des organischen Lösungsmittels verbleibende Rückstand wird aus Ethanol umkristallisiert.

Die Ausbeute beträgt ca. 90 %

Schmp.: 118-119°C; HPLC-Reinheit 99 %.

**Beispiel 2**

(±)-Asocainol-Phosphat

38 g (0,091 Mol) (±)-Asocainol-Base werden unter Erwärmen in 940 ml Ethanol gelöst. Anschließend wird eine Lösung 8,91 g (0,091 Mol) Phosphorsäure in 18 ml Ethanol unter Rühren zugetropft. Nach dem Abkühlen wird der entstandene Niederschlag isoliert.

Ausbeute: 36 g (76,7 % d. Th.)

Schmp.: 147,2°C; HPLC-Reinheit > 99 %

**Beispiel 3**

(±)-Asocainol-Citrat

50 g (0,12 Mol) (±)-Asocainol-Base werden bei Raumtemperatur in 2,5 l Diethylether gelöst. Unter Rühren wird eine Lösung von 25,22 g (0,12 Mol) Citronensäure-Monohydrat in 100 ml Ethanol zugetropft. Der entstandene Niederschlag wird abgesaugt und getrocknet.

Ausbeute: 73 g (99,7) % d. Th.)

Schmp. : 93°C; HPLC-Reinheit > 99 %

**Patentansprüche**

1. Verfahren zur Herstellung von racemischem Asocainol durch Umsetzung von natürlichem Thebain mit Phenethyl-magnesiumhalogenid und Trennung der erhaltenen Diastereomeren (R, R und (R, S) in an sich bekannter Weise durch fraktionierte Kristallisation der Hydrochloride aus einem niederen Alkohol, dadurch gekennzeichnet, daß man das isolierte Diastereomere (R, S) durch Erhitzen auf 138 - 200°C oder als Lösung in einem höher siedenden inerten Lösungsmittel auf 130-200°C teilweise in die (S, S)-Form umlagert, das durch Umlagerung gebildete (S, S)-Enantiomere in an sich bekannter Weise vom unveränderten (R, S) Diastereomeren abtrennt, das (S, S)-Enantiomere im Molverhältnis 1 : 1 mit (+)-Asocainol der (R, R)Form als Base oder Salz in einem Lösungsmittel löst und das gebildete Racemat der (R, R; S,S)-Form in Form seiner Base oder von dessen pharmakologisch verträglichen Salzen isoliert.

2. Verfahren nach Anspruch 1, dadurch gekennzeichnet, daß die thermische Umlagerung in einem höher siedenden inerten organischen Lösungsmittel durchgeführt wird.

**Claims**

1. Process for the preparation of racemic asocainol by reaction of natural thebaine with phenethyl magnesium halide and separation in per se known manner of the diastereomers (R, R) and (R, S) obtained by fraction crystallisation of the hydrochlorides from a lower alcohol, characterised in that one partly rearranges into the (S, S)-form the isolated diastereomer (R, S) by heating to 138 - 200°C. or a solution in a higher boiling inert solvent to 130 - 200°C., separates off in per se known manner the (S, S)-enantiomer thus formed by rearrangement from the unchanged (R, S)-diastereomer, dissolves the (S,S)-enantiomer in the molar ratio of 1 : 1 with (+)-asocainol of the (R, R)-form as base or salt in a solvent and isolates the racemate formed of the (R, R; S, S)-form in the form of its base or of its pharmacologically compatible salts.

2. Process according to claim 1, characterised in that the thermal rearrangement is carried out in a high boiling inert organic solvent.

**Revendications**

1. Procédé de préparation d'asocaïnol racémique par réaction de thébaïne naturelle avec un halogénure de phénéthyl-magnésium et séparation des diastéréoisomères (R, R) et (R, S) obtenus, d'une façon connue en soi, par cristallisation fractionnée des chlorhydrates dans un alcool inférieur, caractérisé en ce que l'on transforme le diastéréoisomère isolé (R, S), par chauffage à une température de 138-200°C, ou sous forme d'une solution dans un solvant inerte de point d'ébullition élevé, à une température de 130-200°C, partiellement en la forme (S, S) , en ce que l'on sépare d'une façon connue en soi l'énantiomère (S, S) formé par transposition du diastéréoisomère (R, S) non transformé, en ce que l'on dissout dans un solvant, dans un rapport molaire 1/1, l'énantiomère (S, S) et le (+)-isocaïnol de forme (R, R) sous forme de base ou de sels, et en ce que l'on isole le racémate (R, R; S, S) obtenu sous forme de base ou d'un de ses sels pharmacologiquement compatibles.

2. Procédé selon la revendication 1, caractérisé en ce que la transposition thermique est mise en oeuvre dans un solvant organique inerte de point d'ébullition élevé.